# EUROPEAN PATENT APPLICATION

(11) **EP 1 811 818 A2**
(43) Date of publication of application: **25.07.2007**
(21) Application number: 07250235.4
(22) Date of filing: 20.01.2007
(51) Int. Cl.: H05B 41/34, A61B 18/20, A61N 5/06

(54) **Improvements in and relating to intense pulsed light devices**

(30) Priority: 21.01.2006 GB 0601222
(71) Applicant: Energist LImited, Swansea SA6 8QY (GB)
(72) Inventor: Davies, Geoffrey Clive, Cardiff CF23 5PH (GB); Howells, Colin Francis, Cwmavon Port Talbot SA12 9AN (GB)
(74) Representative: Lambert, Ian Robert

(57) **Abstract**

A method of calibrating a discharge lamp assembly (5, 6, 9) of an IPL device (1a, 1b) includes the steps of determining the light energy output from the lamp assembly and comparing it with the required target energy output to produce one or more correction factors relative thereto, storing the or each correction factor, and when operating the IPL device, utilising the or each correction factor to adjust the electrical energy supplied to the lamp (5) to thereby effect lamp discharge within the required light output energy range. IPL apparatus is also provided which includes means for determining the light energy output from the discharge lamp assembly, either directly or indirectly, such as by measurement or calculation, to produce a correction factor, means for storing the correction factor and means when operating the IPL device for utilising the correction factor to adjust the electrical energy supplied to the discharge lamp to keep the discharge within the require light output energy range.

## Description

This invention relates to intense pulsed light (IPL) devices of the type which are typically used for hair depilation and skin repair by the well known expedient of subjecting the skin and/or hair follicles to short bursts of high energy light of, typically, 6-55 J/cm². Such devices often take the form of a housing containing a capacitor bank which supplies high voltage electrical energy to a xenon flash lamp contained within a handheld applicator connected to the housing, thereby causing the lamp to discharge light energy through an optical waveguide or window opening to e.g. skin onto which the head of the applicator has been placed, usually through the intermediary of an optical filter, such as a band-pass or cut-off filter, which thereby ensures that only useful wavelengths of light and thus energy are incident on the skin.

A problem with such IPL devices is that they can burn skin unless the type and amount of light energy is carefully controlled so that the device emits sufficient light energy to perform the function required of it, such as hair depilation, whilst allowing a sufficient safety margin to minimise the risk of burning of the skin. Since various parts of the IPL device can contribute to variations in light energy output, such as the length or thickness of the xenon tube, the thickness or purity of the optical filter, the surface reflectance of the lamp reflector, the various tolerances required for each are cumulative and can mean an unacceptably high failure rate once the IPL device has been fully assembled and tested for final use.

The foregoing problems have been addressed in part in our GB2389536B, in which an adjustable light coupler is provided between the xenon lamp and the output window of the handheld applicator which takes advantage of the well known principle that the intensity of light is inversely proportional to the square of the distance from its source. In this way, even relatively large cumulative errors in manufacture which would otherwise render the apparatus either too dangerous for use against the skin due to excess light energy or be ineffective against the skin due to insufficient light energy, can be accommodated by the simple expedient of adjusting the distance of the light source to the output window so that output energy falls within the required target energy range. However, a disadvantage of this system is that, as will be apparent, it requires recalibration every time parts of the system are changed e.g. if a xenon lamp is changed or if a filter is changed, and so on. This may be time consuming and/or require the presence of specially trained personnel and specialist equipment, such that it may even be more economic and/or convenient to simply measure the actual light energy output from a xenon lamp, reflector and associated optical filter (collectively hereafter referred to as 'discharge lamp assembly') and discard those with output energies which fall outside a required energy range.

It is therefore an object of the present invention to address the foregoing problems by providing methods and apparatus whereby substantially all or most of the aforementioned disadvantages are obviated.

According to a first aspect of the invention there is provided a method of calibrating a discharge lamp assembly (as defined) of an IPL device for subsequent discharge within a required light output energy range, the method including the steps of determining the light energy output from the lamp assembly and comparing it with a required target energy output to produce one or more correction factors relative thereto, storing the or each correction factor, and when operating the IPL device, utilising the or each correction factor to adjust the electrical energy supplied to the lamp to thereby effect lamp discharge within the required light output energy range.

Conveniently, the light energy output from the lamp assembly is determined by direct or calculated measurement thereof, preferably during manufacture of the discharge lamp assembly, although instead it may be calculated, such as by calculation of the electrical energy dissipated between the start and finish of an IPL cycle leading to discharge of the discharge lamp. Accordingly, with either or both methods of determining the actual or calculated light energy output from the discharge lamp, a suitable correction factor stored in e.g. non-volatile (permanent) memory during initialisation of the IPL device in which it is fitted, means that, whatever the actual or calculated variation in light output energy from the discharge lamp from the required light output energy range, such can always be adjusted by reference to the correction factor, by suitable adjustment of the electrical power levels to the discharge lamp, such that, in turn, relatively tight control of the light output energy can be achieved.

In accordance with a second aspect of the invention, there is provided IPL apparatus which includes means for determining the light energy output from the discharge lamp assembly for said IPL apparatus, either directly or indirectly, such as by measurement or calculation, to produce a correction factor relative thereto, means for storing said correction factor, and means when subsequently operating said IPL device for utilising the correction factor for the discharge lamp to adjust the electrical energy supplied to it to thereby effect lamp discharge within the required light output energy range.

Conveniently, the discharge lamp assembly is in the form of a cartridge which includes a lamp, such as a xenon lamp, reflector means such as a metallic reflector and filter means, such as an optical filter, which cartridge may conveniently be cooled such as by water cooling. The utilisation of a cartridge having the aforesaid characteristics is particularly convenient since it may be easily replaced after use, such as after 10,000 cycles of use, with each such cartridge having its own unique correction factor determined from a combination of the efficiency of the lamp source, the filter, the reflector and any other parameters which ultimately affect the output energy level achieved.

Most conveniently, the cartridge containing the discharge lamp assembly and associated means for storing the correction factor therefor is included or includable within the applicator itself. Alternatively, the IPL device, if separate from the applicator, may be provided with separate means for determining the energy output from the discharge lamp assembly and thereafter providing a suitable correction factor to the electrical energy input into the device to thereafter ensure light output energy within a required range even if the discharge lamp would otherwise be considered unsuitable for use in a conventional IPL device.

The invention will now be described, with reference to the accompanying drawings, in which:
Figure 1 is a simplified schematic view of a discharge lamp assembly for an IPL apparatus according to the invention,
Figure 2 is a graph showing tolerance variations between standard IPL cartridge-type discharge lamp assemblies before and after calibration in accordance with the invention,
Figure 3 is a schematic view of a method of calibrating the cartridged discharge lamp assembly of Figure 1 in accordance with a first aspect of the invention, and
Figure 4 is a schematic view of a method of operating an IPL device fitted with a calibrated cartridge of Figure 1.

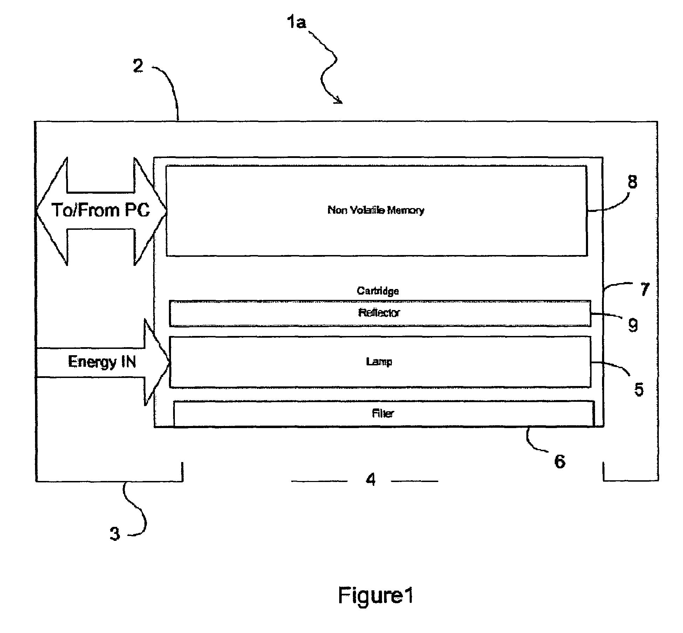

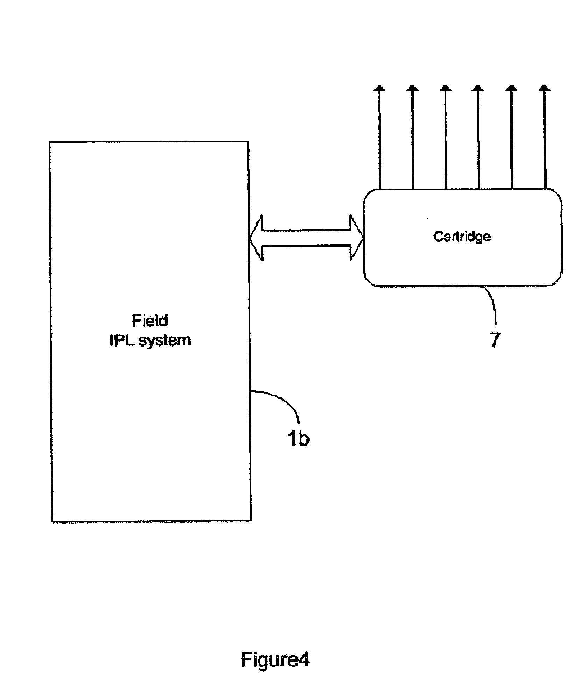

Turning firstly to Figure 1 there is shown in simplified schematic form part of an initial, calibration, IPL device 1a comprising a hollow head 2 which normally forms part of a hand-held applicator (not shown). The head 2 includes a window frame portion 3 defining a window opening 4 through which light from a xenon lamp 5 may pass via an optical filter 6, such as a band-pass or cut-off filter. The lamp 5, filter 6 and reflector 9 together define a "discharge lamp assembly" and are contained within a replaceable cartridge 7 which also includes a non-volatile memory 8. The memory 8 stores non-unique information such as the number of times the lamp 5 has been fired and unique information about the physical and optical properties of the lamp 5, the reflector 9 and filter 6, each of which unique and non-unique information may be accessed as required from a remote, field, IPL device 1b (shown in Figure 4) in a manner to be described.

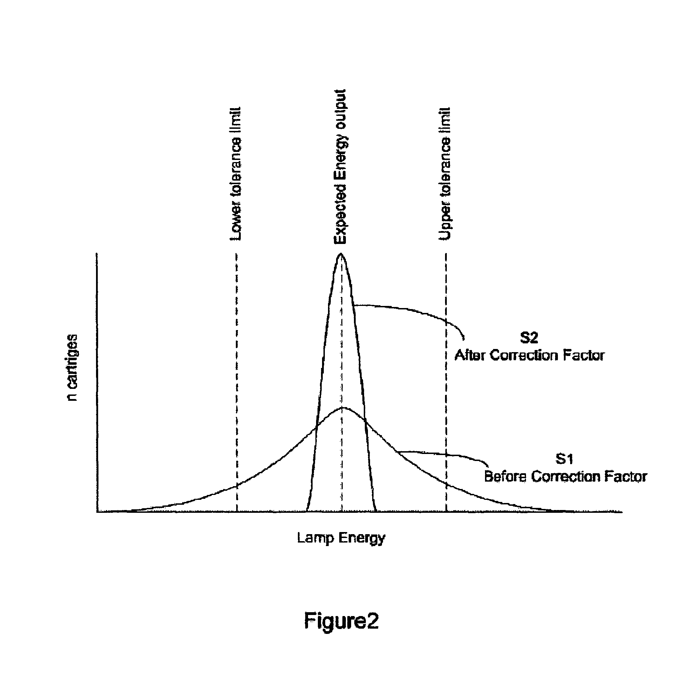

Turning now to Figure 2 there is shown a graph showing a typical spectrum S₁ of measured lamp emitted light energy against "n" newly manufactured and tested cartridges 7, which each include a lamp 5, filter 6 and reflector 9 as shown in Figure 1. As to be expected, because of variations in the measured properties of the lamp 5, filter 6 and reflector 9, which can include the type of materials from which they are made, impurities therein, variations in thickness, length, and so on, the spectra is generally quite flat and spread out over a useable range between a lower tolerance limit and an upper tolerance limit, between which limits the cartridges 7 are considered acceptable for use, and non-useable range where a significant proportion fall on either side of these tolerance limits and which are, ordinarily, simply discarded as being commercially and/or practically useless.

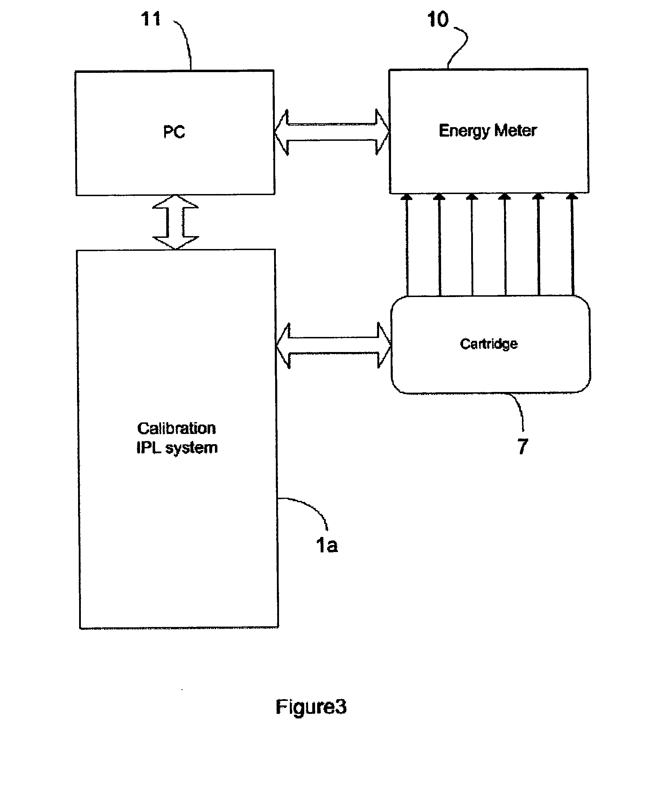

In contrast, as shown in spectrum S₂ and as is discussed in more detail with reference to Figure 3, by applying a correction factor the spectrum S₂ can be made much sharper, leading to essentially all of the cartridges being considered commercially and practically useable, with each being given an assigned correction factor unique to that particular cartridge. This correction factor is applied during manufacture/initial assembly of the cartridge 7 and can thereafter be interrogated by an IPL device 1b (shown in Figure 4).

Figure 3 schematically shows a method of initially calibrating the cartridge 7. Following manufacture of the cartridge 7 its efficiency in terms of light output (shown in multiple arrows) is measured by e.g. a photo electric energy meter 10 which is then interrogated by a PC 11 which compares the measured reading for that particular cartridge 7 to an expected/required energy level to thereafter calculate a correction factor by which the correct energy level may be achieved for that particular cartridge by the simple expedient of increasing the electrical energy supplied to it. The PC 11 therefore provides a correction factor to the calibration IPL device 1a which thereafter sends a calibration signal to the non-volatile memory 8 of the cartridge 7 with its own unique, permanent, correction factor so that when the cartridge 7 is inserted for use within a different, field, IPL system 1b as shown in Figure 4 having its own interrogation capability (not shown) the amount of electrical energy needed to be supplied to the cartridge 7 to produce light output within the required energy range can be automatically calculated and achieved. As a consequence, when the cartridge 7 is first installed within the field IPL device 1b the electrical energy to the lamp 5 is adjusted automatically or manually so that light emanating from the window opening 4 (shown in Figure 1) is at an energy level which falls within the required operational range and safety limits.

By each cartridge 7 also including within the memory 8 a token counter this can also not only be used to indicate how near the cartridge 7 is to reaching its maximum safe usage in terms of lamp usage cycles, typically ten thousand such cycles, but it can also be used to indicate a safe upper limit for use of the cartridge 7, within which limit it will still perform satisfactorily in terms of energy output within the required safety margins.

Although this interrogation of the non-volatile memory 8 may conveniently be from a unique non-volatile memory stored within the cartridge 7 it will, however, be understood that the discharge lamp assembly defined by the lamp 5 filter 6 and reflector 9 may instead be separately provided with its own, unique, correction factor, which may simply be a separately accessible memory chip, or even a look-up or machine readable unique identification number, such as in barcode form, so that an IPL device, when fitted with the discharge lamp assembly 5, 6, 9 can then be supplied with the unique correction factor by which safe operation can be assured by suitable adjustment to the electrical energy supplied to the lamp 5.

Although the establishment of a unique correction factor for individual sets of lamps 5, filters 6 and reflectors 9 during manufacture can be measured by reference to the components shown in Figure 1, it is possible to measure individual components independently, such as the brightness of the lamp 5, the translucency of the filter 6, the reflectance of a lamp reflector 9, and so on, to thereby collectively calculate a combined unique correction factor which may thereafter be used with such components. However, it will be understood that this solution is less attractive to the components being ready assembled into a cartridge and the correction factor then calculated in one operation, since otherwise variables, which may include the distance between the lamp and the filter and the lamp and the reflector, may substantially alter the actual light output from the total calculated by such cumulative measurement techniques.

## Claims

1. A method of calibrating a discharge lamp assembly (as defined) (5, 6, 9) of an IPL device (1a, 1b) for subsequent discharge within a required light output energy range, the method including the steps of determining the light energy output from the lamp assembly and comparing it with a required target energy output to produce one or more correction factors relative thereto, storing the or each correction factor, and when operating the IPL device, utilising the or each correction factor to adjust the electrical energy supplied to the lamp to thereby effect lamp discharge within the required light output energy range.

2. A method according to Claim 1 further **characterised in that** the light energy output from the lamp assembly is determined by direct measurement thereof.

3. A method according to Claim 1 further **characterised in that** the light energy output from the lamp assembly is determined by calculation thereof.

4. A method according to any preceding Claim further **characterised in that** the light energy output from the lamp assembly is determined during manufacture of the discharge lamp assembly.

5. A method according to any one of Claims 1 to 3 further **characterised in that** the light energy output from the lamp assembly is calculated.

6. A method according to Claim 5 further **characterised in that** the light energy output is determined by calculation of the electrical energy dissipated between the start and finish of an IPL cycle leading to discharge of the discharge lamp.

7. IPL apparatus (1a, 1b) which includes means for determining the light energy output from the discharge lamp assembly (5, 6, 9) for said IPL apparatus, either directly or indirectly, such as by measurement or calculation, to produce a correction factor relative thereto, means for storing said correction factor, and means when subsequently operating said IPL device for utilising the correction factor for the discharge lamp to adjust the electrical energy supplied to it to thereby effect lamp discharge within the required light output energy range.

8. Apparatus according to Claim 7 further **characterised in that** the discharge lamp assembly (5, 6, 9) is in the form of a cartridge (7) which includes a lamp (5), such as a xenon lamp, reflector means (9) such as a metallic reflector and filter means (6), such as an optical filter.

9. Apparatus according to Claim 8 further **characterised in that**, during use, the cartridge (7) is cooled, such as by water cooling.

10. Apparatus according to Claim 8 or Claim 9 further **characterised in that** the cartridge (7) containing the discharge lamp assembly (5, 6, 9) and associated means for storing the correction factor therefor is included or includable within a hand-held applicator (2).

11. Apparatus according to any one of Claims 7 to 10 further **characterised in that** the IPL device (1a, 1b) is provided with separate means for determining the energy output from the discharge lamp assembly and thereafter providing a suitable correction factor to the electrical energy input into the device to thereafter ensure light output energy within a required range.
